# EUROPEAN PATENT APPLICATION

(11) **EP 3 851 020 A1**
(43) Date of publication of application: **21.07.2021**
(21) Application number: 21151928.5
(22) Date of filing: 15.01.2021
(51) Int. Cl.: A61B 1/00, A61B 3/00, A61B 90/20

(54) **DIGITAL MAGNIFYING DEVICE**

(30) Priority: 17.01.2020 IT 202000000874
(71) Applicant: Insmile S.r.l., 40127 Bologna (IT)
(72) Inventor: CALICETI, Filippo, 40127 Bologna (IT)
(74) Representative: Maccagnan, Matteo

(57) **Abstract**

A digital magnifying device having a cranial support (3) wearable on the head (2) of a user, a supporting body (4) connected to the cranial support (3), a digital screen (5) mounted on a first side (6) of the supporting body (4), a digital video camera (7) mounted on a second side (8) of the supporting body (4) opposite to the first side (6), and an image processing unit (11). The supporting body (4) has an arm (9) connected to the cranial support (3) so that, in use, the digital video camera (7) frames the space in front of the user's head (2) and the digital screen (5) is arranged in front of the user's eyes at a certain distance therefrom. The image processing unit (11) is configured to magnify and automatically focus images captured by the digital video camera (7) and to command the digital screen (5) so as to display in real-time the magnified images.

## Description

### CROSS-REFERENCE TO RELATED APPLICATIONS

This patent application claims priority from Italian patent application no. 102020000000874 filed on 17/01/2020.

### TECHNICAL FIELD

The present invention relates to a digital magnifying device wearable on the head of a user.

In particular, the present invention is advantageously but not exclusively applied to the medico-surgical field, in particular to the dental field, to which the following description will explicitly refer without thereby losing generality.

### BACKGROUND ART

In dentistry it is standard procedure to use optical magnifying devices, known in general as magnifying binoculars, which enable the user wearing them (typically a doctor) to have a magnified view of a zone of intervention, for example during a surgical intervention or of dental hygiene.

An optical magnifying device of the type used in dentistry comprises a binocular lenses system mounted on a classic glasses frame or on a cranial support. The binocular lenses system has means for the manual focus. Such optical magnifying device enables the user to see in a magnified manner those details of the zone of intervention which would not be distinguishable to the naked eye in a clear manner.

The type of optical magnifying device described above, although greatly widespread, has remarkable drawbacks. First of all, the optical magnifying device has to be worn with the binocular lenses system very close to the eyes of the operator and thus does not enable the latter to have a side and bottom view such to ease the interaction with the surrounding environment, for example for picking up or laying down other intervention instruments. Furthermore, once focused the zone of intervention, the operator should keep the eyes always at the same distance from the work zone so as not to perceive a decrease in sharpness of what seen through the binocular lenses system. Finally, the aforementioned type of optical magnifying devices is heavy and uncomfortable to wear.

### DISCLOSURE OF INVENTION

The object of the present invention is to provide a magnifying device, which is exempt from the above-described drawbacks and, at the same time, is easy and cost-effective to manufacture.

In accordance with the present invention, a digital magnifying device is provided according to what claimed in the appended claims.

### BRIEF DESCRIPTION OF THE DRAWINGS

To better understand the present invention, a preferred embodiment is described in the following, by way of nonlimiting example and with reference to the accompanying drawings, wherein:
- Figure 1 illustrates a three-quarter front perspective view of the digital magnifying device object of the present invention; and
- Figure 2 illustrates the digital magnifying device of Figure 1 according to a three-quarter rear perspective view.

### BEST MODE FOR CARRYING OUT THE INVENTION

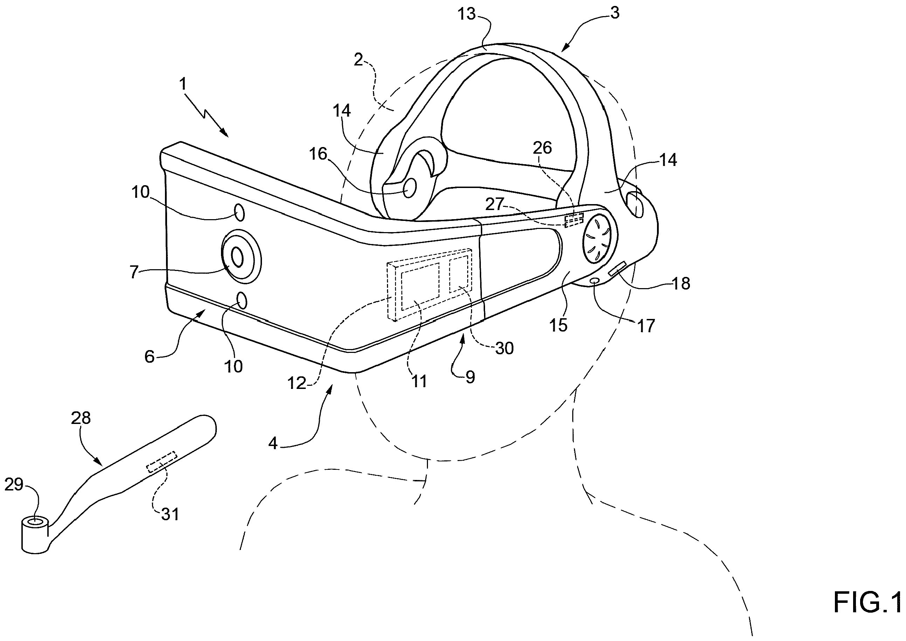

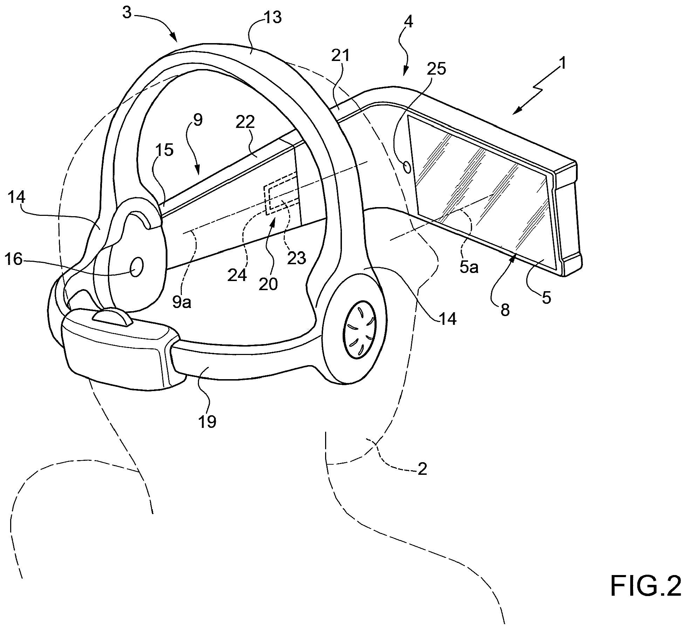

In Figures 1 and 2, reference numeral 1 generically indicates, as a whole, the digital magnifying device of the present invention. The device 1 is shown when being worn by a user, in particular when it is fitted on the head 2 of the user.

The device 1 comprises a cranial support 3 wearable on the head 2 of the user, a supporting body 4 connected to the cranial support 3, a digital screen 5 (Figure 2) mounted on a first side 6 (Figure 2) of the supporting body 4, at least one digital video camera 7 mounted on a second side 8 (Figure 1) of the supporting body 4 opposite to the side 6. The supporting body 4 comprises an arm 9, which is connected to the cranial support 3 so that, in use, the video camera 7 frames the space in front of the user's head 2 and the screen 5 is arranged in front of the user's eyes (not visible in Figure) at a certain distance therefrom.

With particular reference to Figure 1, the device comprises at least one light source 10 arranged on the side 8 for illuminating the space framed by the video camera 7. The light source 10 comprises one or more white light LEDs. In the example of Figure 1, two light sources 10 are present arranged one above and the other below the video camera 7.

The device 1 comprises an image processing unit 11, which is configured to magnify and automatically focus images captured by the video camera 7 and to command the screen 5 so as to display in real-time the magnified images. In the example illustrated by Figure 1, the arm 9 comprises an inner cavity 12 and the image processing unit 11 is preferably housed in the cavity 12. The image processing unit 11 comprises a video card for the control of the screen 5.

The cranial support 3 comprises an upper arch 13, which comprises two end portions 14 adapted to be arranged, in use, at the two ears of the user, and the arm 9 comprises a connection end 15, which is connected to one of the two end portions 14 so as to enable the user a side view, from the opposite side to that of the arm 9, and a bottom view of the surrounding environment. In the example of Figures 1 and 2, the arm 9 is connected to the end portion 14 which is at the left ear of the user so as to enable the latter a side view from the right side.

Each of the end portions 14 of the upper arch 13 is provided with a respective earphone 16 for listening to audio signals. The two acoustic earphones 16 are connected to a female jack connector 17 arranged in one of the two end portions 14.

The cranial support 3 comprises at least one USB socket 18 arranged in one of the two end portions 14.

With particular reference to Figure 2, the cranial support 3 comprises an adjustable nape band 19.

In all, the cranial support 3 comprises four or six rests on the cranial circumference.

Always with reference to Figure 2, the arm 9 has a longitudinal axis 9a, preferably transverse to a plane orthogonal to a central vision axis 5a of the screen 5, and comprises an adjustment system 20 for adjusting its length along the longitudinal axis 9a in order to adjust the distance between the screen 5 and the user's eyes. In the example of Figure 2, the screen 5 is rectangular and flat and thus the central vision axis 5a is orthogonal to the entire plane of the screen 5.

In particular, the arm 9 is divided in two longitudinal portions 21 and 22 connected to one another in a movable manner by means of the adjustment system 20. The portion 21 is integral with the screen 5 and the portion 22 comprises the connection end 15. The adjustment system 20 comprises, for example, a slide 23 integral with the portion 21 and stick-slip sliding in a guide 24 integral with the portion 22.

The arm 9 has a minimum length such that, in use, the distance between the screen 5 and the user's eyes is adjustable from a minimum value at least equal to 10 cm. Figure 2 illustrates the arm 9 in the minimum distance configuration.

The device 1 comprises a microphone 25 arranged on the side 6 of the supporting body 4 so as to be, in use, facing the user's face. The microphone 25 is connected to the female jack connector 17 by means of a small electric cable (not illustrated) which runs inside the arm 9.

The connection end 15 of the arm 9 is releasably connected to one of the end portions 14 of the upper arch 13. In particular, the connection end 15 of the arm 9 comprises first mechanical connection means (not illustrated) and each of the two end portions 14 of the upper arch 13 comprises second mechanical connection means (not illustrated) releasably coupled to the first mechanical connection means so that the arm 9 can be mounted as required on any one of the two end portions 14.

For such reason, the supporting body 4 has a symmetric structure with respect to a plane on which both the longitudinal axis 9a of the arm 9 and the central vision axis 5a of the screen 5 lie.

By way of example, the first mechanical connection means comprise a non-through hole and the second mechanical connection means comprise a pin adapted to engage with interference in the hole, or vice versa the first mechanical connection means comprise a pin and the second mechanical connection means comprise a hole engageable with interference by the pin.

With reference again to Figure 1, the connection end 15 of the arm 9 comprises a series of electric contacts 26 connected to the image processing unit 11 by means of an electric cable (not illustrated) and each of the two end portions 14 of the upper arch 13 comprises another series of electric contacts 27 adapted to come into contact with the series of electric contacts 26 when the first mechanical connection means are coupled to the second mechanical connection means.

The series of electric contacts 27 of each of the two end portions 14 is connected to the USB socket 18. In this manner, the image processing unit 11 is communicatively connectable with a personal mobile communication device (not illustrated) of the user, for example a smartphone, so as to enable the recording and the remote storing of videos or images captured with the video camera 7 and the remote connection with the zone of intervention by a doctor not present in the room where the user wearing the device 1 is present. The user and the doctor in remote connection can dialogue with one another thanks to the earphones 16 and to the microphone 25 connectable to the personal mobile communication device by means of the female jack connector 17.

To such purpose, on the personal mobile communication device a software application is installed designed to control the image processing unit 11 so as to activate/deactivate the video camera 7 and the screen 5, activate the storing of static images and/or videos on the mobile communication device or on a remote server, or establish an audio/video streaming connection with a remote display and communication unit at the disposal of a doctor who is in a place distant from the zone of intervention, such remote display and communication unit being for example implemented by a further mobile communication device (smartphone or PC tablet). The audio part of the streaming connection is for example bidirectional so as to incorporate the audio signals generated by the microphone 25 and the audio signals transmitted to the earphones 16 and the video part of the streaming connection is for example unidirectional so as to incorporate the video signal generated by the video camera 7.

Furthermore, the coupling between the series of electric contacts 26 and the series of electric contacts 27 enables using the personal mobile communication device as electric power supply for the electronic modules on board the supporting body 4, i.e. the screen 5, the video camera 7 and the image processing unit 11. To such purpose, in the arm 9 an electric wiring is present (not illustrated) connected to the series of electric contacts 26 so as to absorb an electric current of power supply from the USB socket 18 and provide it directly to the various electronic modules or so as to electrically power the screen 5 and the video camera 7 through the image processing unit 11.

The coupling between the series of electric contacts 26 and the series of electric contacts 27 also enables the connection between the female jack connector 17 and the microphone 25. In other words, the microphone 25 is connected to at least one contact of the series of electric contacts 26 and the female jack connector 17 is connected to at least one contact of the series of electric contacts 27 of each of the two end portions 14.

The device 1 further comprises a wireless communication module 30, which is communicatively connected to the image processing unit 11 and, is preferably housed in the cavity 12 of the arm 9, and a handpiece 28, which comprises a further digital video camera 29 adapted to take macro shots and a wireless communication module 31 communicatively connected to the video camera 29 and adapted to communicate with the wireless communication module 30 in order to produce a communication connection between the video camera 29 and the image processing unit 11. The handpiece 28 has the shape of a dental technology handpiece. The image processing unit 11 is configured to automatically focus images captured by the video camera 29 and to command the screen 5 so as to display in real-time the images of the video camera 29 in combination with the images of the video camera 7 according to the known picture in picture (PIP) technique.

Preferably, the video part of the aforementioned streaming connection comprises a combination of the images of the video camera 29 with those of the video camera 29 according to the same PIP layout displayed on the screen 5.

Preferably, the wireless communication modules 30 and 31 are composed of Bluetooth communication modules.

According to a further embodiment not illustrated of the present invention, the supporting body 4 differs from the one illustrated by Figures 1 and 2 due to the fact of comprising an arm different from the arm 9 and in particular connected in a non-releasable manner to a central portion of the upper arch 13 of the cranial support 3 so as to enable the user a complete side view, i.e. both to the right and to the left.

While the above-described invention particularly refers to a very precise embodiment example, it is not to be understood as limited to such embodiment example, falling within its scope all those variants, modifications or simplifications claimed in the appended claims, such as for example:
- the screen 5 is curved, in particular concave; and
- the device 1 comprises two or three video cameras 7 arranged on the side 6 of the supporting body 4 and adapted to take simultaneous shots with different depths of field, such simultaneous shots being combined by the image processing unit 11 before being displayed on the screen 5 so as to improve the perception of depth by the user.

The main advantage of the above-described device 1 is to enable the user a side and bottom view of the surrounding environment and it simultaneously enables an automatic focus of the zone of intervention without the user having to move his/her head in order to see where to lay down or from where to pick up the instruments necessary for the intervention, and/or having to manually focus the device if the head moves back from or comes too near the framed subject. Furthermore, the device 1, when connected to a personal mobile communication device by means of the USB socket 18, enables a doctor to remotely connect so as to see to zone of intervention and guide the user during the intervention.

## Claims

1. A digital magnifying device comprising a cranial support (3) wearable on the head (2) of a user, a supporting body (4) connected to the cranial support (3), a digital screen (5) mounted on a first side (6) of the supporting body (4), at least one digital video camera (7) mounted on a second side (8) of the supporting body (4) opposite to the first side (6), and image processing means (11); the supporting body (4) comprising an arm (9), which is connected to the cranial support (3) so that, in use, the digital video camera (7) frames the space in front of the user's head (2) and the digital screen (5) is arranged in front of the user's eyes at a certain distance therefrom; the image processing means (11) being configured to magnify and automatically focus first images captured by the digital video camera (7) and to command the digital screen (5) so as to display in real-time the first magnified images.

2. The digital magnifying device according to claim 1, wherein said cranial support (3) comprises an upper arch (13), which comprises two end portions (14) adapted to be arranged, in use, at the two ears of the user, and said arm (9) comprises a connection end (15), which is connected to one of the two end portions (14) in such a way as to enable the user at least a side view, from the opposite side to that of the arm (9), of the surrounding environment.

3. The digital magnifying device according to claim 2, wherein said connection end (15) of the arm (9) comprises first mechanical connection means and each of said two end portions (14) of the upper arch (13) comprises second mechanical connection means which can be releasably coupled to said first mechanical connection means so that the arm (9) can be mounted as required on any one of the two end portions (14).

4. The digital magnifying device according to claim 3, wherein said connection end (15) of the arm (9) comprises a first series of electric contacts (26) connected to said image processing means (11) and each of said two end portions (14) of the upper arch (13) comprises a second series of electric contacts (27) adapted to come into contact with the first series of electric contacts (26) when said first mechanical connection means are coupled to said second mechanical connection means; the cranial support (3) comprising at least one USB socket (18) connected to the second series of electric contacts (27) of at least one of the two end portions (14).

5. The digital magnifying device according to claim 4, and comprising an electric wiring arranged in said arm (9) and connected to said first series of electric contacts (26) to absorb an electric current from said USB socket (18) and distribute it so as to electrically power said image processing means (11), said digital screen (5) and said digital video camera (7).

6. The digital magnifying device according to any one of the claims from 2 to 5, wherein each of said end portions (14) of the upper arch (13) is provided with a respective earphone (16).

7. The digital magnifying device according to any one of the claims from 1 to 6, wherein said arm (9) has a longitudinal axis (9a), preferably transverse to a plane orthogonal to a central vision axis (5a) of the digital screen (5), and comprises adjustment means (20) to adjust its length along said longitudinal axis (9a) in order to adjust the distance between the digital screen (5) and the user's eyes.

8. The digital magnifying device according to claim 7, wherein said arm (9) has a minimum length such that, in use, the distance between the digital screen (5) and the user's eyes is adjustable from a minimum value at least equal to 10 cm.

9. The digital magnifying device according to any one of the claims from 1 to 8, and comprising at least one light source (10) arranged on said second side (8) to illuminate the space framed by said at least one digital video camera (7) .

10. The digital magnifying device according to any one of the claims from 1 to 9, and comprising a microphone (25) arranged on said first side (6).

11. The digital magnifying device according to any one of the claims from 1 to 10, wherein said arm (9) comprises an inner cavity (12) and said image processing means (11) are housed in said inner cavity (12).

12. The digital magnifying device according to any one of the claims from 1 to 11, and comprising a first wireless communication module (30), which is arranged in said arm (9) and communicatively connected to said image processing means (11), and a handpiece (28), which comprises a further digital video camera (29) adapted to take macro shots and a second wireless communication module (31) communicatively connected to said further digital video camera (29) and adapted to communicate with the first wireless communication module (30); said image processing means (11) being configured to automatically focus second images captured by the further digital video camera (29) and to command the digital screen (5) so as to display in real-time the second images in combination with the first images according to a picture in picture technique.
